**Europäisches Patentamt**

**European Patent Office**

**Office européen des brevets**

(19)

(11) Veröffentlichungsnummer: **0 064 284**
A2

(12) ## EUROPÄISCHE PATENTANMELDUNG

(21) Anmeldenummer: **82103688.6**

(22) Anmeldetag: **30.04.82**

(51) Int. Cl.³: **A 61 M 25/00**

(30) Priorität: **02.05.81 DE 3117416**

(43) Veröffentlichungstag der Anmeldung:
**10.11.82 Patentblatt 82/45**

(84) Benannte Vertragsstaaten:
**AT BE CH DE FR GB IT LI LU NL SE**

(71) Anmelder: **Nessler, Reiner, Dr.**
**Paracelsiusstrasse 24**
**D-3320 Salzgitter-Bad(DE)**

(72) Erfinder: **Nessler, Reiner, Dr.**
**Paracelsiusstrasse 24**
**D-3320 Salzgitter-Bad(DE)**

(74) Vertreter: **Suchy, Herbert**
**Byk Gulden Lomberg Chemische Fabrik GmbH**
**Patentabteilung Byk-Gulden-Strasse 2**
**D-7750 Konstanz(DE)**

(54) **Venenkatheter.**

(57) Es wird ein Venenkatheter zur Messung des zentralen Venendrucks in der oberen Hohlvene vorgeschlagen, bestehend aus einem in die Vene einzuführenden flexiblen Kunststoffschlauch, der an seinem einen Ende in ein Ansatzstück größeren Durchmessers übergeht und in diesem Bereich eine Fixationsplatte trägt, die in der Schlauchebene liegt und den Kunststoffschlauch beidseitig überragt, der sich dadurch auszeichnet, daß an dem Ansatzstück Verbindungsmittel vorgesehen sind, die ein Befestigen bzw. Lösen der Fixationsplatte in Richtung senkrecht zur Schlauchebene ermöglichen. Die Handhabung von herkömmlichen Venenkathetern wird damit verbessert, indem es nunmehr möglich ist, bei angelegtem Venenkatheter die verschmutzte Fixationsplatte nach oben abzuziehen und durch eine neue Fixationsplatte zu ersetzen.

Fig. 1

EP 0 064 284 A2

## Venenkatheter

## Technisches Gebiet

Die Erfindung betrifft einen Venenkatheter zur Messung des zentralen Venendrucks in der oberen Hohlvene, bestehend aus einem in die Vene einzuführenden flexiblen Kunststoffschlauch, der an seinem einen Ende in ein Ansatzstück größeren Durchmessers übergeht und in diesem Bereich eine Fixationsplatte trägt, die in der Schlauchebene liegt und den Kunststoffschlauch beidseitig überragt.

## Stand der Technik

Eine derartige Ausführungsform läßt sich dem deutschen Gebrauchsmuster 80 06 872 entnehmen. Die Fixationsplatte besteht bei diesem vorbekannten Venenkatheter aus Schaumgummi. Die Fixationsplatte weist eine mittlere Durchtrittsöffnung auf, mit der die Fixationsplatte auf den Kunststoffschlauch aufgezogen ist.

Dieser Venenkatheter hat sich in der Praxis bewährt, weist jedoch einen Nachteil auf. Die Fixationsplatte kann insbesondere bei längerer Verwendung durch Blut und/oder Flüssigkeit verschmutzen, so daß es aus Gründen der Sterilität wünschenswert wäre, diese Fixationsplatte auszuwechseln. Dies ist aber nicht möglich, da die Fixationsplatte auf den Kunststoffschlauch aufgezogen ist, der Katheter aber zum Auswechseln der Fixationsplatte nicht wieder aus der Vene herausgezogen werden kann.

Der Erfindung liegt somit die Aufgabe zugrunde, die Handhabung des eingangs erläuterten Venenkatheters zu verbessern.

## Darstellung der Erfindung

Diese Aufgabe wird erfindungsgemäß dadurch gelöst, daß an dem Ansatzstück Verbindungsmittel vorgesehen sind, die ein Befestigen bzw. Lösen der Fixationsplatte in Richtung senkrecht zur Schlauchebene ermöglichen.

Durch diese Ausbildung ist es nunmehr möglich, bei angelegtem Venenkatheter die verschmutzte Fixationsplatte nach oben abzuziehen und
durch eine neue Fixationsplatte zu ersetzen.

Dabei ist es zweckmäßig, wenn die Verbindungsmittel mit der Fixationsplatte einen Formschluß in Richtung der Schlauchachse bilden. Während
bei dem vorbekannten Venenkatheter bei entsprechend starkem Zug in
Schlauchrichtung ein Herausziehen des Kunststoffschlauches durch die
Fixationsplatte hindurch möglich war, wird dies aufgrund des in der
Schlauchachse wirksamen Formschlusses zwischen Fixationsplatte und
Ansatzstück verhindert.

In einer besonders einfach herzustellenden Ausführungsform können die
Verbindungsmittel einstückig mit dem Ansatzstück ausgebildete Aufdrückknacken sein, denen in der Fixationsplatte entsprechende Ausnehmungen
zugeordnet sind. Die Fixationsplatte selbst ist zweckmäßig streifenförmig ausgebildet, weist eine ausreichende Elastizität auf und besteht
vorzugsweise ebenfalls aus Kunststoff. Die Fixationsplatte wird also
einfach auf die genannten Knacken aufgedrückt, wobei aufgrund der Elastizität der Fixationsplatte und/oder der Aufdrückknacken eine selbsttätige Verriegelung erfolgt. Wenn in der Fixationsplatte eine im
Querschnitt angenähert halbkreisförmig ausgebildete Nut zur Aufnahme
des Ansatzstückes vorgesehen wird, schmiegt sich die Fixationsplatte
sehr flach an den Venenkatheter an und läßt sich leicht auf der Haut
des Patienten festlegen.

Um den neuen Venenkatheter einfach, vor allem aber steril in eine bei
dem Patienten bereits eingeführte Punktionskanüle einschieben zu können,
ist es vorteilhaft, wenn in den von seiner Fixationsplatte getrennten
Venenkatheter ein Mandrin eingeschoben wird, der den Venenkatheter an
beiden Enden überragt, und wenn der so bestückte Venenkatheter durch
eine Schutztüte steril umschlossen wird, die an ihrem unteren Ende
einen Einschubkonus aufweist, gegen den das aus dem Kunststoffschlauch
herausragende Ende des Mandrins weist. Nachdem in üblicher Weise aus der
angelegten Punktionskanüle die innere Stahlkanüle herausgezogen wird,
kann die vorstehend genannte Schutztüte mit ihrem Einschubkonus an die
in der Regel aus Plastik bestehende Punktionskanüle angesetzt bzw.
fest mit ihr verbunden werden. Durch Drücken gegen das obere Ende der

S22 EP — 3 — 0064284 4/82

Schutztüte tritt das aus dem Kunststoffschlauch herausragende Ende des Mandrins zuerst in den Einschubkonus der Schutztüte ein und gelangt von dort problemlos in die noch in der Vene befindliche Punktionskanüle. Auf den so eingeführten Mandrin kann dann der Venenkatheter nachgeschoben werden, wobei die Punktionskanüle in geeigneter Weise zerlegt wird. Die Durchführung dieses Verfahrens wird nur dadurch möglich, daß der Venenkatheter getrennt von seiner Fixationsplatte in der Schutztüte verpackt und die Fixationsplatte erst nach Beendigung des Anlegens des Venenkatheters montiert werden kann.

Damit der Mandrin nicht vollständig in den Venenkatheter eintauchen kann, ist es vorteilhaft, wenn das aus dem Ansatzstück herausragende Ende des Mandrins einen Ansatz größeren Durchmessers aufweist. Dabei kann es sich um eine Verdickung des Mandrins, oder aber um ein angebrachtes Arretierstück handeln.

Zur Vermeidung einer Thrombose ist es wünschenswert, die Außenwandung des Kunststoffschlauches des Venenkatheters von Zeit zu Zeit mit einer Flüssigkeit zu benetzen, die die Blutgerinnung innerhalb der Vene verringert und dadurch die Thrombosegefahr herabsetzt. Deshalb kann erfindungsgemäß vorgesehen werden, daß der Kunststoffschlauch in seinem für die Einführung in die Vene bestimmten Bereich Perforationslöcher aufweist, deren Öffnungsquerschnitt kleiner ist als der des Kunststoffschlauches. Diese Perforationslöcher dürfen lediglich in dem Bereich des Kunststoffschlauches vorgesehen werden, der mit Sicherheit innerhalb der Vene, nicht aber mehr im Gewebe liegt. Die Dimensionierung der Perforationslöcher wird so gewählt, daß ihr Durchströmwiderstand größer ist als der Ausströmwiderstand am Ende des Katheterschlauches. Durch einen derartigen, sogenannten einlumigen Katheterschlauch kann dann die Infusionsflüssigkeit oder aber auch Blut in üblicher Weise eingeführt werden, wobei aufgrund der genannten Dimensionierung keine oder nur geringfügige Anteile Flüssigkeit durch die Perforationsöffnungen austreten. Zum Benetzen der Katheteraußenwandung wird dann eine gerinnungshemmende Flüssigkeit unter Druck eingespritzt, die dann aus den Perforationsöffnungen austritt.

Die dem Ansatzstück des Venenkatheters am nächsten liegenden Perforationslöcher können eine gegen das Ansatzstück gerichtete Ausströmrichtung aufweisen. Dadurch läßt sich auch der Bereich der Außenwandung
des Kunststoffschlauches bespülen, der zwar bereits innerhalb der Vene
liegt, aus Sicherheitsgründen aber noch frei von Perforationsöffnungen
ist.

Bei mehrlumigen Katheterschläuchen kann auch der die übrigen Lumina
umschließende Schlauch perforiert und dann ausschließlich für die Zufuhr der gerinnungshemmenden Flüssigkeit vorgesehen werden.

Der neue Venenkatheter eignet sich nicht nur für die Einführung in Venen
sondern auch in Arterien.

In der Zeichnung ist eine als Beispiel dienende Ausführungsform der Erfindung schematisch dargestellt. Es zeigen:

    Fig. 1    einen in einer Schutztüte verpackten Venenkatheter
             und

    Fig. 2    eine dem Venenkatheter gemäß Fig. 1 zugeordnete
             Fixationsplatte.

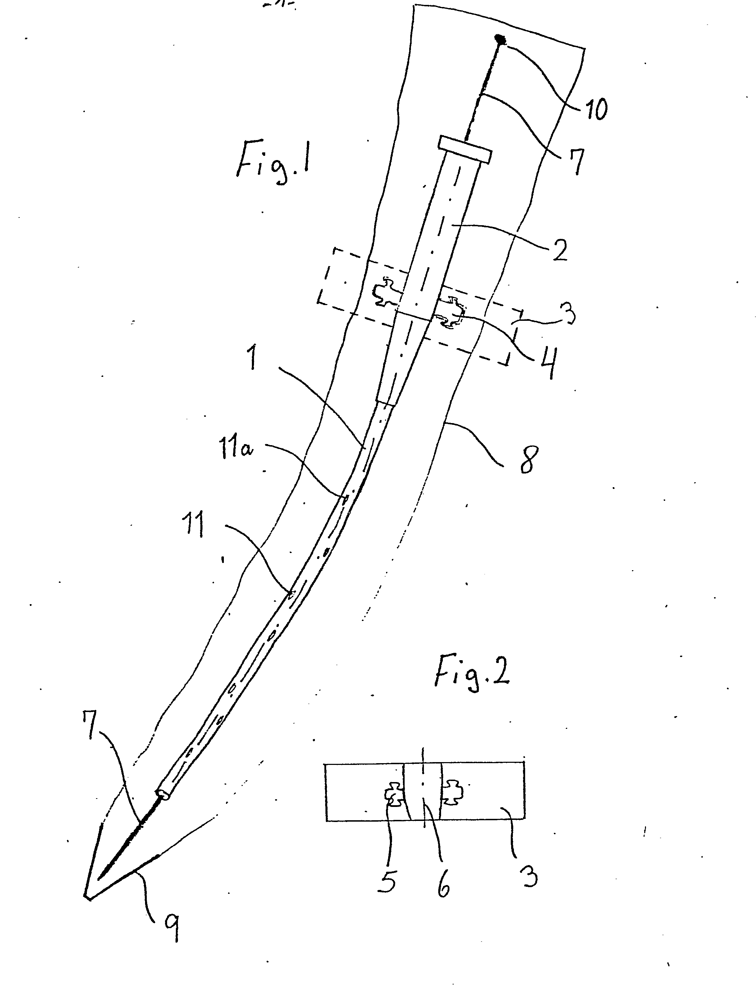

Gemäß Fig. 1 besteht der dargestellte Venenkatheter aus einem in die
Vene einzuführenden flexiblen Kunststoffschlauch 1, dessen eines Ende
in ein Ansatzstück 2 größeren Durchmessers übergeht. An dem Ansatzstück 2 läßt sich eine Fixationsplatte 3 lösbar befestigen. Hierfür
sind Verbindungsmittel 4 vorgesehen, die bei dem dargestellten Ausführungsbeispiel aus einstückig mit dem Ansatzstück 2 ausgebildeten
Aufdrückknacken bestehen, denen in der Fixationsplatte 3 entsprechende Ausnehmungen 5 zugeordnet sind. Außerdem ist in der Fixationsplatte
3 eine im Querschnitt angenähert halbkreisförmig ausgebildete Nut 6
zur Aufnahme des Ansatzstückes 2 vorgesehen. Diese in Fig. 2 in Draufsicht dargestellte Fixationsplatte 3 läßt sich, nachdem sie so um $180^{0}$
gedreht ist, daß die Nut 6 auf dem Ansatzstück 2 aufliegt, auf die Verbindungsmittel 4 des Venenkatheters von oben, also senkrecht zur
Schlauchebene aufdrücken und würde dann die in Fig. 1 gestrichelt dargestellte Lage einnehmen.

In den Venenkatheter ist ein Mandrin 7 eingeschoben, der den Venenkatheter an beiden Enden überragt. Venenkatheter mit eingefädeltem Mandrin sind in einer Schutztüte 8 verpackt, die an ihrem unteren Ende einen Einschubkonus 9 aufweist. Damit der Mandrin 7 nicht vollständig in den Venenkatheter hineinrutschen kann, weist er an seinem oberen Ende einen Ansatz 10 größeren Durchmessers auf.

Der Kunststoffschlauch 1 weist in seinem für die Einführung in eine Vene oder Arterie bestimmten Bereich Perforationslöcher 11 auf, deren Öffnungsquerschnitt kleiner ist als der des Kunststoffschlauches 1. Dabei weisen die dem Ansatzstück 2 am nächsten liegenden Perforationslöcher 11a eine gegen das Ansatzstück 2 gerichtete Ausströmrichtung auf.

Patentansprüche

1. Venenkatheter zur Messung des zentralen Venendrucks in der oberen Hohlvene, bestehend aus einem in die Vene einzuführenden flexiblen Kunststoffschlauch, der an seinem einen Ende in ein Ansatzstück größeren Durchmessers übergeht und in diesem Bereich eine Fixationsplatte trägt, die in der Schlauchebene liegt und den Kunststoffschlauch beidseitig überragt, dadurch gekennzeichnet, daß an dem Ansatzstück (2) Verbindungsmittel (4) vorgesehen sind, die ein Befestigen bzw. Lösen der Fixationsplatte (3) in Richtung senkrecht zur Schlauchebene ermöglichen.

2. Venenkatheter nach Anspruch 1, dadurch gekennzeichnet, daß die Verbindungsmittel (4) mit der Fixationsplatte (3) einen Formschluß in Richtung der Schlauchachse bilden.

3. Venenkatheter nach Anspruch 1 oder 2, dadurch gekennzeichnet, daß die Verbindungsmittel (4) einstückig mit dem Ansatzstück (2) ausgebildete Aufdrückknacken sind, denen in der Fixationsplatte (3) entsprechende Ausnehmungen (5) zugeordnet sind.

4. Venenkatheter nach Anspruch 1, 2 oder 3, dadurch gekennzeichnet, daß in der Fixationsplatte (3) eine im Querschnitt angenähert halbkreisförmig ausgebildete Nut (6) zur Aufnahme des Ansatzstückes (2) vorgesehen ist.

5. Venenkatheter nach einem der vorhergehenden Ansprüche, gekennzeichnet durch einen eingeschobenen, den von seiner Fixationsplatte (3) getrennten Venenkatheter an beiden Enden überragenden Mandrin (7) und durch eine Venenkatheter mit Mandrin (7) steril umschließende Schutztüte (8), die an ihrem unteren Ende einen Einschubkonus (9) aufweist, gegen den das aus dem Kunststoffschlauch (1) herausragende Ende des Mandrins (7) weist.

6. Venenkatheter nach Anspruch 5, dadurch gekennzeichnet, daß das aus dem Ansatzstück (2) herausragende Ende des Mandrins (7) einen Ansatz (10) größeren Durchmessers aufweist.

7. Venenkatheter nach einem der vorhergehenden Ansprüche, dadurch gekennzeichnet, daß der Kunststoffschlauch (1) in seinem für die Einführung in die Vene bestimmten Bereich Perforationslöcher (11) aufweist, deren Öffnungsquerschnitt kleiner ist als der des Kunststoffschlauches (1).

8. Venenkatheter nach Anspruch 7, dadurch gekennzeichnet, daß die dem
   Ansatzstück (2) am nächsten liegenden Perforationslöcher (11a) eine
   gegen das Ansatzstück (2) gerichtete Ausströmrichtung aufweisen.

0064284

Fig.1

Fig.2

Dr. Reiner Nessler
3320 Salzgitter-Bad